# EUROPEAN PATENT APPLICATION

(11) **EP 4 428 164 A1**
(43) Date of publication of application: **11.09.2024**
(21) Application number: 22890280.5
(22) Date of filing: 27.10.2022
(51) Int. Cl.: C08F 6/00, C08F 14/06, B01D 53/14, B01D 53/04

(54) **METHOD AND APPARATUS FOR RECOVERING UNREACTED MONOMER**

(30) Priority: 03.11.2021 KR 20210149849
(71) Applicant: Hanwha Solutions Corporation, Jung-gu Seoul 04541 (KR)
(72) Inventor: LIM, Dongwook, Daejeon 34128 (KR); LEE, Sangwook, Daejeon 34128 (KR); LEE, Shinbeom, Daejeon 34128 (KR)
(74) Representative: Berggren Oy
(86) International application number: PCT/KR2022/016625
(87) International publication number: WO 2023/080545

(57) **Abstract**

The present invention relates to a method and an apparatus for recovering an unreacted monomer and, more specifically, to a method for recovering an unreacted monomer, in which economic efficiency is increased by efficiently increasing the recovery rate of an unreacted monomer. The method for recovering an unreacted monomer, according to the present invention, comprises the steps of: (S1) supplying an exhaust gas discharged from a reactor and an absorbent to an absorption unit, and absorbing an unreacted monomer into the absorbent; (S2) transferring, to a heat exchange unit, the absorbent into which the unreacted monomer has been absorbed and heating same; (S3) supplying the heated absorbent and a heating medium to a stripping unit, and stripping and recovering the unreacted monomer from the absorbent; (S4) transferring, to the heat exchange unit, the absorbent from which the unreacted monomer has been stripped and cooling same; and (S5) resupplying the cooled absorbent to the absorption unit.

## Description

### [Technical Field]

The present invention relates to a method and device for recovering an unreacted monomer, and more particularly, to a method for recovering an unreacted monomer that increases cost effectiveness by efficiently increasing a recovery rate of an unreacted monomer.

### [Background Art]

PVC is generally produced by suspension polymerization, emulsion polymerization, or bulk polymerization, and is usually produced by the suspension polymerization and the emulsion polymerization, which have the advantages that the heat of reaction may be easily removed and a product of high purity may be obtained.

In the suspension polymerization and the emulsion polymerization, a vinyl chloride monomer (VCM) and a polymerization initiator together with an aqueous medium, a dispersant, and an emulsifier are injected into a polymerization vessel equipped with a stirrer and are stirred, and a temperature inside the polymerization vessel is maintained to polymerize the VCM.

In this case, in general, the polymerization reaction does not continue until the VCM is entirely converted into PVC and is terminated at a polymerization conversion rate with high production efficiency. An unreacted monomer (VCM) remaining in gas discharged after termination is a carcinogen and is subject to emissions regulations under the Clean Air Conservation Act of Korea because it is harmful.

Accordingly, the VCM is recovered through several recovery methods as described in Korean Patent Publication No. 10-0266479 "Adsorption type capture and recovery device for volatile petroleum compound" and Korean Patent Publication No. 10-0582718 "Method and device for continuous vacuum thermal regeneration of adsorbent and separation and recovery of adsorption material".

The conventional VCM recovery process is performed through absorption, adsorption, pressure swing adsorption (PSA), a membrane, and the like, and in practice, in commercial use, the VCM is recovered through a secondary adsorption process after a primary absorption process.

However, in the VCM recovery method described above, the amount of VCM absorbed into an absorbent during the absorption process is not large, which increases the load of the adsorption process and reduces a replacement cycle of the adsorbent, thereby reducing process efficiency. In addition, since activated carbon, which is a commonly used adsorption material, does not have a high adsorption rate, when the VCM is not sufficiently absorbed during the absorption process, the amount of VCM emitted into the atmosphere increases as the VCM is supplied beyond adsorption capacity of the activated carbon, which may easily violate emission concentration regulations.

### [Related Art Document]

(Patent Document 1) Korean Patent Publication No. 10-0266479
(Patent Document 2) Korean Patent Publication No. 10-0582718

### [Disclosure]

### [Technical Problem]

An object of the present invention is to provide a method and device for recovering an unreacted monomer that increases cost effectiveness by efficiently increasing a recovery rate of an unreacted monomer.

### [Technical Solution]

In one general aspect, a method for recovering an unreacted monomer includes: (S1) supplying an exhaust gas discharged from a reactor and containing an unreacted monomer and an absorbent to an absorption unit to allow the absorbent to absorb the unreacted monomer; (S2) transferring the absorbent into which the unreacted monomer is absorbed to a heat exchange unit and heating the transferred absorbent; (S3) supplying the heated absorbent and a heating medium to a stripping unit and stripping and recovering the unreacted monomer from the absorbent; (S4) transferring the absorbent from which the unreacted monomer is stripped to the heat exchange unit and cooling the transferred absorbent; and (S5) resupplying the cooled absorbent to the absorption unit.

In the method for recovering an unreacted monomer according to an embodiment of the present invention, the absorbent may contain a plasticizer.

In the method for recovering an unreacted monomer according to an embodiment of the present invention, the absorbent may be derived from the following Chemical Formula 1.

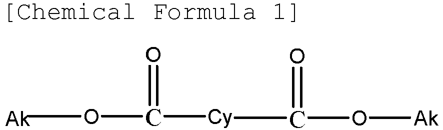

(In Chemical Formula 1, Cy is phenylene or cyclohexylene, and Ak is a C₃ to C₁₀ linear or branched alkyl group.)

In the method for recovering an unreacted monomer according to an embodiment of the present invention, the absorbent may be dioctyl terephthalate (DOTP) or bis (2-ethylhexyl) cyclohexane-1,4-dicarboxylate (DEHCH).

In the method for recovering an unreacted monomer according to an embodiment of the present invention, the step (S1) may be performed at a temperature of 20 to 50°C and a pressure of 1 to 4 barg.

In the method for recovering an unreacted monomer according to an embodiment of the present invention, in the step (S2), a temperature of the absorbent heated by the heat exchange unit may be 90 to 120°C.

In the method for recovering an unreacted monomer according to an embodiment of the present invention, in the step (S3), a temperature of the heating medium may be 120 to 200°C.

The method for recovering an unreacted monomer according to an embodiment of the present invention may further include (Sl-A) supplying and adsorbing a residual unreacted monomer that is not absorbed into the absorbent in the absorption unit to an adsorption unit.

In the method for recovering an unreacted monomer according to an embodiment of the present invention, in the step (S1), each of the exhaust gas and the absorbent may be supplied to have a counter-current flow in the absorption unit.

In the method for recovering an unreacted monomer according to an embodiment of the present invention, in the step (S3), each of the absorbent and the heating medium may be supplied to have a counter-current flow in the stripping unit.

In the method for recovering an unreacted monomer according to an embodiment of the present invention, the unreacted monomer may be a vinyl chloride monomer (VCM).

In another general aspect, a device for recovering an unreacted monomer includes an absorption tower to which each of an exhaust gas discharged from a reactor and an absorbent is supplied and in which an unreacted monomer is absorbed into the absorbent; a first recovery line for recovering the absorbent into which the unreacted monomer is absorbed from the absorption tower; a first heat exchanger that heats the absorbent supplied from the first recovery line; a second recovery line for recovering the heated absorbent from the first heat exchanger; a stripping tower to which each of the absorbent supplied from the second recovery line and a heating medium is supplied and in which the unreacted monomer is stripped from the absorbent; a first circulation line for recovering the absorbent from which the unreacted monomer is stripped from the stripping tower; a second heat exchanger that cools the absorbent supplied from the first circulation line; and a second circulation line for resupplying the cooled absorbent from the second heat exchanger to the absorption tower.

In the device for recovering an unreacted monomer according to an embodiment of the present invention, the absorbent may be a plasticizer for a vinyl chloride polymer, and the unreacted monomer may be a vinyl chloride monomer (VCM) .

In the device for recovering an unreacted monomer according to an embodiment of the present invention, the first and second heat exchangers may be integrated.

In the device for recovering an unreacted monomer according to an embodiment of the present invention, the first heat exchanger may further include a heater, and the second heat exchanger may further include a cooler.

In the device for recovering an unreacted monomer according to an embodiment of the present invention, the absorption tower may include an absorption body, a first supply port formed at a lower portion of the absorption body and supplying the exhaust gas into the absorption body, a second supply port located at an upper portion of the absorption body and supplying the absorbent into the absorption body, a first discharge port formed at a bottom of the absorption body and discharging the absorbent into which the unreacted monomer is absorbed, and a second discharge port formed at a top of the absorption body and discharging an unreacted monomer that is not absorbed into the absorbent.

In addition, the stripping tower may include a stripping body, a third supply port formed at a lower portion of the stripping body and supplying the heating medium into the stripping body, a fourth supply port located at an upper portion of the stripping body and supplying the heated absorbent into the stripping body, a third discharge port formed at a bottom of the stripping body and discharging the absorbent from which the unreacted monomer is stripped, and a fourth discharge port formed at a top of the stripping body and discharging the unreacted monomer stripped from the absorbent.

The device for recovering an unreacted monomer according to an embodiment of the present invention may further include an adsorption tower connected to a top of the absorption tower and adsorbing an unreacted monomer that is not absorbed into the absorbent and is discharged from the absorption tower.

The device for recovering an unreacted monomer according to an embodiment of the present invention may further include a third heat exchanger connected to a top of the stripping tower and separating condensed water from the unreacted monomer discharged from the stripping tower.

### [Advantageous Effects]

The method for recovering an unreacted monomer according to the present invention may increase a recovery rate by recovering the unreacted monomer through an absorbent that may absorb and strip the unreacted monomer.

In addition, as the absorbent may be circulated and reused, process efficiency may be maximized and process costs may be reduced.

### [Description of Drawings]

FIG. 1 is a schematic view of an absorption device according to an embodiment of the present invention.
FIG. 2 illustrates a graph showing a change in recovery rate according to repetition of VCM absorption and stripping according to a VCM recovery method according to an embodiment of the present invention.

### [Best Mode]

Unless otherwise defined, all the technical terms and scientific terms used in the present specification have the same meanings as commonly understood by those skilled in the art to which the present invention pertains. The description for the known function and configuration unnecessarily obscuring the gist of the present invention will be omitted in the following description and the accompanying drawings.

In addition, unless the context clearly indicates otherwise, the singular forms used in the present specification may be intended to include the plural forms.

In addition, units used in the present specification without special mention are based on weight, and as an example, a unit of % or a ratio means wt% or a weight ratio. Unless otherwise defined, wt% means wt% of any one component accounted for in the total composition.

In addition, a numerical range used in the present specification includes upper and lower limits and all values within these limits, increments logically derived from a form and span of a defined range, all double limited values, and all possible combinations of the upper and lower limits in the numerical range defined in different forms. Unless otherwise specifically defined in the specification of the present invention, values out of the numerical range that may occur due to experimental errors or rounded values also fall within the defined numerical range.

In the present specification, the expression "comprise(s)" is intended to be an open-ended transitional phrase having an equivalent meaning to "include(s)", "contain(s)", "have (has)", and "is (are) characterized by", and does not exclude elements, materials, or steps, all of which are not further recited herein.

A method for recovering an unreacted monomer of the present invention includes: (S1) supplying an exhaust gas discharged from a reactor and an absorbent to an absorption unit to allow the absorbent to absorb an unreacted monomer; (S2) transferring the absorbent into which the unreacted monomer is absorbed to a heat exchange unit and heating the transferred absorbent; (S3) supplying the heated absorbent and a heating medium to a stripping unit and stripping and recovering the unreacted monomer from the absorbent; (S4) transferring the absorbent from which the unreacted monomer is stripped to the heat exchange unit and cooling the transferred absorbent; and (S5) resupplying the cooled absorbent to the absorption unit, and in a conventional method for recovering an unreacted monomer, which is a harmful substance, during an absorption process, the amount of harmful substance absorbed into an absorbent is not large, which increases a load of an adsorption process, reduces a replacement cycle of the adsorbent, and reduces process efficiency. In addition, since activated carbon, which is a commonly used adsorption material, does not have a high adsorption rate, when the monomer is not sufficiently absorbed during the absorption process, the amount of VCM emitted into the atmosphere increases as the VCM is supplied beyond adsorption capacity of the activated carbon, which may easily violate emission concentration regulations.

However, in the method for recovering an unreacted monomer of the present invention, the recovery rate of the monomer may be efficiently increased and a concentration of the monomer discharged may be minimized, and when a subsequent adsorption process is performed, the load of the adsorbent is reduced and the replacement cycle of the adsorbent is significantly increased, which may significantly increase the cost effectiveness of the process. In addition, the process costs may be further reduced by circulating the absorbent through an absorbent that may absorb and strip a monomer.

In addition, in the present invention, as a specific absorbent that has a significantly high absorption degree of an unreacted monomer is used, an unreacted monomer may be recovered at a high recovery rate with only one recovery process without the need to repeatedly recover an unreacted monomer.

In the present invention, the method for recovering an unreacted monomer is a method for recovering an unreacted monomer in an exhaust gas by absorbing and stripping the unreacted monomer into and from an absorbent, and specifically, may be a VCM absorption and stripping method for recovering a volatile substance containing a vinyl chloride monomer (VCM), but is not limited thereto.

Specifically, in the step (S1) of supplying the exhaust gas discharged from the reactor and the absorbent to the absorption unit to allow the absorbent to absorb the unreacted monomer, a gaseous exhaust gas and a liquid absorbent are respectively supplied to an absorption unit in which an accommodation space is formed and then brought into contact with each other, such that the unreacted monomer is absorbed into the absorbent.

The exhaust gas is discharged from suspension polymerization and emulsion polymerization processes and refers to a form in which an unreacted monomer is present inside. For example, the exhaust gas may refer to a gas that is discharged from the reactor after polyvinyl chloride (PVC) is subjected to suspension polymerization in the reactor and contains a VCM therein, but is not limited thereto.

The unreacted monomer refers to a harmful substance generated during the suspension polymerization and emulsion polymerization processes, and a specific example thereof includes a vinyl chloride monomer (VCM).

The absorption unit is not limited as long as it provides an accommodation space in which the exhaust gas and the absorbent may come into contact with each other, and in detail, the absorption unit may be an absorption tower known in the art.

The absorbent may be a plasticizer, and may be preferably a plasticizer for a vinyl chloride polymer. More preferably, the absorbent may be derived from a monomer of the following Chemical Formula 1.

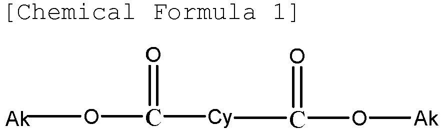

(In Chemical Formula 1, Cy is phenylene or cyclohexylene, and Ak is a C₃ to C₁₀ linear or branched alkyl group.)

More preferably, the absorbent may be dioctyl terephthalate (DOTP) or bis(2-ethylhexyl) cyclohexane-1,4-dicarboxylate (DEHCH). Since the absorbent has a significantly high absorption rate of an unreacted monomer including a vinyl chloride monomer (VCM), the absorbent has a high removal rate of the unreacted monomer through absorption, and at the same time, has excellent stripping ability under certain conditions, such that a stripping recovery rate of the absorbent may be significantly high. Accordingly, an unreacted monomer may be efficiently recovered. Specifically, the VCM may be recovered in an amount of 95% or more, and more specifically, 99% or more, with respect to the total amount of VCM contained in the exhaust gas. In addition, such an absorbent may maintain a high recovery rate even when repeatedly absorbing and stripping the unreacted monomer at a high temperature, which is significantly advantageous in terms of process efficiency.

The step (S1) is not limited as long as the absorption of the unreacted monomer through the absorbent is possible, and may be performed at a temperature of 20 to 50°C, and specifically, 30 to 40°C, and a pressure of 1 to 4 barg, and specifically, 2 to 3.5 barg. Under the conditions in the above ranges, the amount of unreacted monomer absorbed is high compared to the amount of absorbent added.

In the step (S1), each of the exhaust gas and the absorbent may be supplied to have a counter-current flow in the absorption unit. Accordingly, gas-liquid (exhaust gas-absorbent) contact may be optimally achieved, and the process efficiency may be further increased.

The step (S2) of transferring the absorbent into which the unreacted monomer is absorbed to the heat exchange unit and heating the transferred absorbent is a step of primarily providing thermal energy so that the unreacted monomer may be stripped from the absorbent in the stripping tower.

The heat exchange unit is provided for heating the absorbent, a heat exchanger known in the art may be used, and the absorbent may be heated through a calorific value of the absorbent from which the unreacted monomer is stripped, which will be described below, and an additionally provided heater.

In the step (S2), a temperature of the absorbent heated by the heat exchange unit may be 80 to 150°C, and specifically, 90 to 120°C. The heating may be efficiently performed within the above ranges.

The step (S3) is a step of supplying the absorbent heated by the heat exchange unit and the heating medium to the stripping unit and stripping and recovering the unreacted monomer from the absorbent, and as the liquid absorbent and the gaseous heating medium come into contact with each other and thermal energy is supplied from the heating medium to the absorbent, the unreacted monomer may be stripped and recovered from the absorbent.

The heating medium may be gas or vapor, and specifically, steam (water vapor). A temperature of the heating medium is not limited as long as it is above a boiling point of the unreacted monomer so that the unreacted monomer may be volatilized and stripped from the absorbent, and preferably, the temperature of the heating medium may be 130 to 200°C. In the above range, the VCM may be recovered at a high stripping rate compared to a calorific value of the heating medium added.

In the step (S3), each of the absorbent and the heating medium may be supplied to have a counter-current flow in the stripping unit. Accordingly, gas-liquid (heating medium-absorbent) contact may be optimally achieved, and the process efficiency may be further increased.

In the step (S4) of transferring the absorbent from which the unreacted monomer is stripped to the heat exchange unit and cooling the transferred absorbent and the step (S5) of resupplying the cooled absorbent to the absorption unit, as a certain amount of absorbent is circulated and used in the recovery process, there is no need to add an additional absorbent, and the unreacted monomer may be recovered more cost-effectively and efficiently.

Specifically, the step (S4) of transferring the absorbent from which the unreacted monomer is stripped to the heat exchange unit and cooling the transferred absorbent is a step of cooling the heated absorbent in the stripping tower so that the unreacted monomer may be reabsorbed into the absorbent. The heat exchange unit is provided for cooling the absorbent, a heat exchanger known in the art may be used, and the absorbent may be cooled through the absorbent into which the unreacted monomer is absorbed described above and an additionally provided cooler.

In the step (S4), a temperature of the absorbent cooled by the heat exchange unit is the absorption performance temperature of the step (S1) described above, and may be 20 to 50°C, and specifically, 30 to 40°C.

The step (S5) is a step of resupplying the absorbent cooled in the step (S4) to the absorption unit and allows the absorbent subjected to absorption and stripping to be reused in the entire recovery process without adding an additional absorbent, and the absorbent subjected to the step (S5) may be used as the absorbent in the step (S1) described above. Such a recovery method allows the absorbent to be circulated and used, such that the process efficiency may be further increased.

In one aspect of the present invention, the method may further include, after the step (S1), (Sl-A) supplying and adsorbing a residual unreacted monomer that is not absorbed into the absorbent in the absorption unit to an adsorption unit. In this case, the adsorption unit may be an adsorption tower known in the art and filled with activated carbon such as activated carbon. The method further includes the adsorption step, such that an unreacted monomer (VCM) emitted into the atmosphere may be further minimized.

A device for recovering an unreacted monomer of the present invention includes an absorption tower to which each of an exhaust gas discharged from a reactor and an absorbent is supplied and in which an unreacted monomer is absorbed into the absorbent; a first recovery line for recovering the absorbent into which the unreacted monomer is absorbed from the absorption tower; a first heat exchanger that heats the absorbent supplied from the first recovery line; a second recovery line for recovering the heated absorbent from the first heat exchanger; a stripping tower to which each of the absorbent supplied from the second recovery line and a heating medium is supplied and in which the unreacted monomer is stripped from the absorbent; a first circulation line for recovering the absorbent from which the unreacted monomer is stripped from the stripping tower; a second heat exchanger that cools the absorbent supplied from the first circulation line; and a second circulation line for resupplying the cooled absorbent from the second heat exchanger to the absorption tower.

Conventionally, the recovery device recovers the unreacted monomer (VCM) by adsorbing the unreacted monomer through an activated carbon tower (adsorption tower). However, as the recovery device as described above recovers the adsorption material (VCM) by adsorbing the adsorption material through activated carbon with a small absorption capacity, it is inconvenient to frequently replace the activated carbon and has the disadvantage of being unsuitable for large-capacity facilities. In addition, the VCM recovery efficiency is low, and thus, the efficiency in operating and maintaining the facility is significantly low.

However, the recovery device of the present invention may recover an unreacted monomer through adsorption and stripping of the unreacted monomer from a specific adsorbent, and may efficiently increase a recovery rate and minimize a concentration of the monomer to be discharged. In addition, the device has a structure that circulates the absorbent, and may continuously adsorb and strip the unreacted monomer without adding an additional adsorbent.

In the present invention, the device for recovering an unreacted monomer is a device for recovering an unreacted monomer in an exhaust gas by absorbing and stripping the unreacted monomer into and from an absorbent, and specifically, may be a VCM absorption and stripping device for recovering a volatile substance containing a vinyl chloride monomer (VCM), but is not limited thereto. Preferably, the device may be a recovery device for performing the method for recovering an unreacted monomer described above.

Hereinafter, the device for recovering an unreacted monomer according to an embodiment of the present invention will be described in detail with reference to the accompanying drawings. The accompanying drawings are provided by way of example in order to sufficiently transfer the spirit of the present invention to those skilled in the art, and the present invention is not limited to the accompanying drawing provided below, but may be implemented in another form.

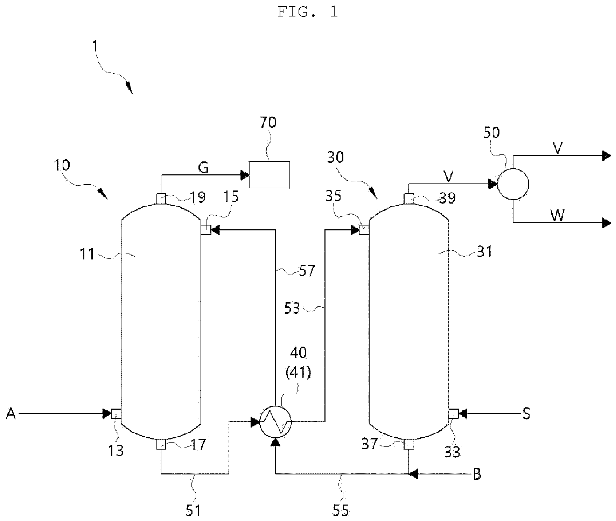

FIG. 1 illustrates a schematic view of a recovery device according to an embodiment of the present invention.

Hereinafter, in the present embodiment, recovery of a vinyl chloride monomer contained in atmospheric exhaust gas generated during a polyvinyl chloride (PVC) production process will be described as an example. Specifically, the unreacted monomer may include a vinyl chloride monomer (VCM).

Referring to FIG. 1, the device for recovering an unreacted monomer of the present invention includes: an absorption tower 10; a first recovery line 51; a first heat exchanger 40; a second recovery line 53; a stripping tower 30; a first circulation line 55; a second heat exchanger 41; and a second circulation line 57.

Specifically, each of an exhaust gas A discharged from a reactor and an absorbent B is supplied and an unreacted monomer is absorbed into the absorbent in the absorption tower 10, and the absorption tower 10 includes an absorption body 11, a first supply port 13 formed at a lower portion of the absorption body 11 and supplying the exhaust gas A into the absorption body 11, a second supply port 15 located at an upper portion of the absorption body 11 and supplying the absorbent into the absorption body 11, a first discharge port 17 formed at a bottom of the absorption body 11 and discharging the absorbent into which the unreacted monomer is absorbed, and a second discharge port 19 formed at a top of the absorption body 11 and discharging an unreacted monomer that is not absorbed into the absorbent.

The absorbent B is a liquid and may be a plasticizer for a vinyl chloride polymer. Specifically, the absorbent B is preferably an absorbent used in the recovery method described above.

The absorption body 11 refers to a body part of the absorption tower 10 that forms an accommodation space, a gas distributor connected to the first supply port 13 may be installed inside the lower portion of the absorption body 11 to distribute the exhaust gas A discharged from the reactor in a PVC production process to the inside of the adsorption body, the VCM may be removed by the adsorbent as the distributed gas flows from the lower portion to the upper portion of the absorption body 11, and a gas G from which the VCM is removed may be discharged through the second discharge port 19 located at the top of the absorption body 11.

A solvent distributor connected to the second supply port 15 may be installed inside the upper portion of the absorption body 11 to distribute the absorbent for VCM absorption into the adsorption body, the distributed liquid absorbent may absorb the VCM as the absorbent flows from the upper portion to the lower portion of the absorption body 11, and the absorbent into which the VCM is absorbed may be discharged through the first discharge port 17 located at the bottom of the absorption body 11.

The absorption body 11 may be divided into a plurality of plates in a length direction to optimize gas-liquid contact, and each plate may be filled with a filler. As an example, the absorption body 11 may be divided into five plates, but is not limited thereto. Each plate may be divided by a grid plate installed horizontally in the absorption body 11, holes formed in the grid plate may be sized so that the filler cannot pass through the holes, and the filler between the respective plates may be filled to fill 70 to 80% of a space volume formed between the respective plates.

For gas-liquid contact, any filler filled in the absorption tower 10 or the adsorption tower in the art may be applied as the filler.

The first recovery line 51 recovers the absorbent into which the unreacted monomer is absorbed from the absorption tower 10, and connects the first discharge port 17 of the absorption tower 10 and the first heat exchanger 40 to be described below to transfer the absorbent into which the VCM is absorbed to the first heat exchanger 40. In the first recovery line 51, a transfer pump may be installed to facilitate the transfer of the absorbent.

The first heat exchanger 40 heats the absorbent supplied from the first recovery line 51, and may be formed integrally with the second heat exchanger 41 to be described below. Specifically, the first and second heat exchangers 40 and 41 are formed integrally between the absorption tower 10 and the stripping tower 30 so that the thermal energy of the adsorbent transferred to the absorption tower 10 and the stripping tower 30 may be exchanged with each other. The first heat exchanger 40 may further include a heater connected in series so that the adsorbent may be further heated and transferred to the stripping tower 30.

The second recovery line 53 recovers the heated absorbent from the first heat exchanger 40, and connects the first heat exchanger 40 and the stripping tower 30 to be described below to transfer the heated absorbent to the stripping tower 30. In the second recovery line 53, a transfer pump may be installed to facilitate the transfer of the absorbent.

Each of the absorbent supplied from the second recovery line 53 and a heating medium S is supplied to the stripping tower 30 to strip an absorption target from the absorbent, and the stripping tower 30 includes a stripping body 31, a third supply port 33 formed at a lower portion of the stripping body 31 and supplying the heating medium S into the stripping body 31, a fourth supply port 35 located at an upper portion of the stripping body 31 and supplying the heated absorbent into the stripping body 31, a third discharge port 37 formed at a bottom of the stripping body 31 and discharging the absorbent from which the unreacted monomer is stripped, and a fourth discharge port 39 formed at a top of the stripping body 31 and discharging the unreacted monomer stripped from the absorbent.

In this case, the heating medium S may be steam (water vapor), but is not limited thereto.

The stripping body 31 refers to a body part of the stripping tower 30 that forms an accommodation space, a distributor connected to the third supply port 33 may be installed inside the upper portion of the stripping body 31 to distribute the heated absorbent to the inside of the stripping body 31, the distributed absorbent may be heated by the heating medium S and the VCM may be stripped as the absorbent flows from the upper portion to the lower portion of the stripping body 31, and the absorbent from which the VCM is stripped may be discharged through the third discharge port 37 located at the bottom of the stripping body 31.

A distributor connected to the third supply port 33 is installed inside the lower portion of the stripping body 31 to distribute the heating medium S for supplying thermal energy to the absorbent into the inside of the stripping body 31, and the distributed heating medium S comes into contact with the absorbent as the heating medium S flows from the lower portion to the upper portion of the stripping body 31, provides thermal energy to the absorbent, and strips the VCM from the absorbent. The stripped VCM (V) may be discharged through the fourth discharge port 39 located at the top of the stripping body 31.

The stripping body 31 may be divided into a plurality of plates in a length direction to optimize gas-liquid contact, and each plate may be filled with a filler. As an example, the stripping body 31 may be divided into seven plates, but is not limited thereto. Each plate may be divided by a grid plate installed horizontally in the stripping body 31, holes formed in the grid plate may be sized so that the filler cannot pass through the holes, and the filler between the respective plates may be filled to fill 70 to 80% of a space volume formed between the respective plates.

For gas-liquid contact, any filler filled in the absorption tower 10 or the adsorption tower in the art may be applied as the filler.

The first circulation line 55 recovers the absorbent from which the unreacted monomer is stripped from the stripping tower 30, connects the third discharge port of the stripping tower 30 and the second heat exchanger 41 to transfer the absorbent from which the VCM is stripped to the second heat exchanger 41. In the second recovery line 53, a transfer pump may be installed to facilitate the transfer of the absorbent.

The second heat exchanger 41 cools the absorbent supplied from the second recovery line 53, and may be formed integrally with the first heat exchanger 40 described above. The second heat exchanger 41 may further include a cooler connected in series so that the adsorbent may be further cooled and transferred to the absorption tower 10.

The second circulation line 57 resupplies the cooled absorbent from the second heat exchanger 41 to the absorption tower 10, and connects the second heat exchanger 41 and the absorption 10 to transfer the cooled absorbent to the absorption tower 10. In the second circulation line 57, a transfer pump may be installed to facilitate the transfer of the absorbent.

In one aspect of the present invention, the device for recovering an unreacted monomer may further include an adsorption tower connected to the second discharge port 19 located at a top of the absorption tower 10 and adsorbing the unreacted monomer (VCM) that is not absorbed into the absorbent and is discharged from the absorption tower 10. The adsorption tower is known in the art, and may be, for example, a multi-plate adsorption tower filled with activated carbon. The recovery device that further includes such an adsorption tower may maximize a VCM recovery rate.

In one aspect of the present invention, the device may further include a water separation unit 50 connected to a top of the stripping tower 30 and separating condensed water from the unreacted monomer discharged from the stripping tower 30. The water separation unit may separate VCM (V) and condensed water W with a heat exchange method, which allows a higher purity VCM to be obtained.

Hereinafter, a recovery process using the present device will be described.

The exhaust gas A discharged from the reactor of the PVC production process is supplied to the inside of a lower portion of the absorption tower 10 through the first supply port 13 of the absorption tower 10 and flows from the lower portion to an upper portion of the absorption tower 10. In this case, the adsorbent B stored in a plasticizer storage tank is supplied to the second supply port 15 formed at the upper portion of the absorption tower 10 through the first and second circulation lines 55 and 57 and flows downward while passing through each plate installed in the absorption tower 10. Accordingly, in the absorption tower 10, the absorbent and the exhaust gas A come into contact with each other, and the VCM contained in the exhaust gas A is adsorbed into the adsorbent. In this case, in the absorption tower 10, the optimal residence time and contact state between gas and liquid are maintained by the filler filled in the absorption tower 10 and each plate, such that the VCM adsorption rate by the adsorbent may be maximized.

As the exhaust gas A moves to the upper portion of the absorption tower 10, the VCM is removed, and the gas G from which the VCM is removed is discharged through the second discharge portion 19 formed at the top of the absorption tower 10.

The adsorbent into which the VCM is adsorbed is transferred to the first heat exchanger 40 through the first recovery line 51, heated, and then transferred to the stripping tower 30 through the second recovery line 53. The heated adsorbent is supplied through the fourth supply port 35 formed at an upper portion of the stripping tower 30 and flows downward while passing through each plate installed in the stripping tower 30. In this case, steam is supplied to the inside of the stripping tower 30 through the third supply port 33 formed at a lower portion of the stripping tower 30, flows from the lower portion to the upper portion of the stripping tower 30, comes into contact with the heated adsorbent, and further heats the heated adsorbent. Accordingly, the VCM (V) is separated from the adsorbent by the boiling point thereof and is stripped from the adsorbent. The optimal residence time and contact state between gas and liquid are maintained due to the filling of each plate of the stripping tower 30, such that the stripping efficiency may be further increased.

The separated VCM (V) is discharged through the fourth discharge port 39 formed at the top of the stripping tower 30.

Meanwhile, the adsorbent separated from the VCM in the stripping tower 30 is supplied to the second circulation line 57 through the third discharge port 37, transferred to the second heat exchanger 41, cooled, and then resupplied to the absorption unit through the first circulation line 55, and then the absorption process is performed again.

In this case, as the first and second heat exchangers 40 and 41 are formed integrally, the absorbent flowing through the first and second recovery lines 51 and 53 may be heated through the thermal energy of the absorbent flowing through the first and second circulation lines 55 and 57, and conversely, the absorbent flowing through the first and second circulation lines 55 and 53 may be cooled by the absorbent flowing through the first and second recovery lines 51 and 53.

Hereinafter, the present invention will be described in more detail with reference to Examples and Comparative Examples. However, the following Examples and Comparative Examples are only examples for describing the present invention in more detail, and the present invention is not limited by the following Examples and Comparative Examples.

### [Example 1]

A VCM recovery process was performed through a recovery device of the present invention illustrated in FIG. 1.

An exhaust gas discharged from a reactor and an absorbent were supplied to an absorption unit at a molar flow ratio of 1:15. Dioctyl terephthalate (DOTP) was used as the absorbent, absorption conditions in an absorption tower were a temperature of 35°C and a pressure of 3 barg, and stripping conditions in a stripping tower were a temperature of 120°C and a pressure of -0.9 barg.

### [Example 2]

A VCM recovery process was performed in the same manner as that of Example 1, except that bis(2-ethylhexyl) cyclohexane-1,4-dicarboxylate (DEHCH) was used as the absorbent.

### [Example 3]

A VCM recovery process was performed in the same manner as that of Example 1, except that the temperature of the stripping condition in the stripping tower was set to 140°C, and the absorption and stripping processes were performed five times.

### [Comparative Example 1]

A VCM recovery process was performed in the same manner as that of Example 1, except that silicon oil was used as the absorbent.

### [Comparative Example 2]

A VCM recovery process was performed in the same manner as that of Comparative Example 1, except that the temperature of the stripping condition in the stripping tower was set to 140°C, and the absorption and stripping processes were performed five times.

Table 1 shows the results of the concentration of VCM absorbed during the absorption process in the absorption tower of each of Examples and Comparative Examples. The VCM concentration was analyzed through gas chromatography.

**[Table 1]**

| Classification | Concentration of VCM absorbed (ppm) |
|---|---|
| Example 1 | 58,485 |
| Example 2 | 55,072 |
| Comparative Example 1 | 38,360 |

Referring to Table 1, it was confirmed that the VCM absorption rate using the absorbent of the present invention was significantly higher than that of silicon oil, which was known as a conventional absorbent.

Table 2 shows the results of measuring the VCM absorption rate, stripping rate, and absorbent loss rate of Example 1 and Comparative Example 1. Specifically, the absorption rate was measured by comparing the concentration of VCM absorbed into the absorbent after the absorption process with the concentration of VCM in the exhaust gas, and the stripping rate was measured by comparing the concentration of VCM remaining in the absorbent after stripping with the concentration of VCM absorbent into the absorbent after the absorption process. In addition, the loss rate of the absorbent was measured by comparing the amount of absorbent after the process with the amount of absorbent added.

**[Table 2]**

| Classification | Example 1 | Comparative Example 1 |
|---|---|---|
| Absorption rate (%) | 99.83 | 99.65 |
| Stripping rate (%) | 99.1 | 99.9 |
| Loss rate (%) | 0.00003 | 0.0002 |

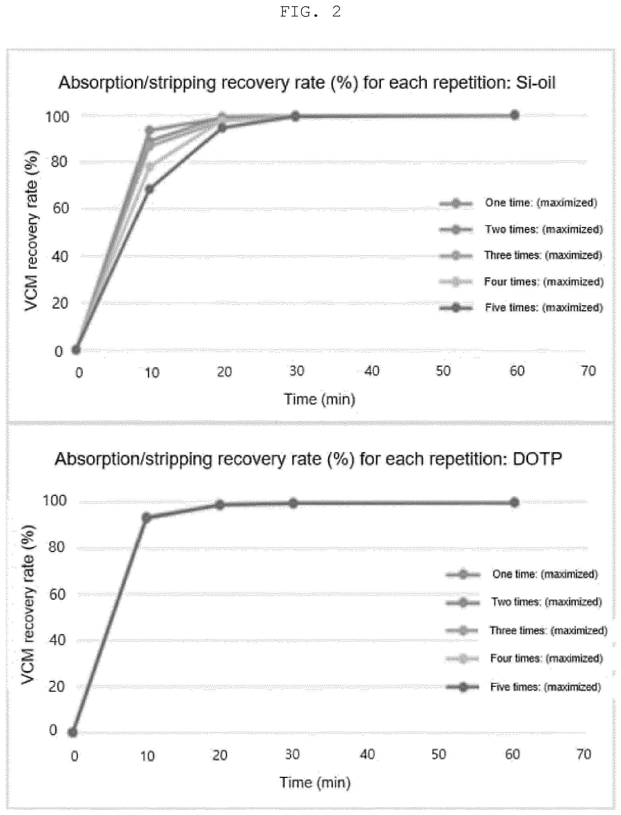

Referring to Table 2, it was confirmed that the VCM recovery efficiency through the absorbent of the present invention was significantly excellent. Specifically, it was confirmed that the absorption rate of Example was superior to that of Comparative Example 1, and the loss rate of the absorbent was also significantly lower than that of Comparative Example. FIG. 2 illustrates a graph showing a change in recovery rate according to the repeated VCM absorption and stripping.

Referring to Table 1 and FIG. 2, it was confirmed that the initial stripping rate of the absorbent of the present invention was slightly lower than that of Comparative Example, but the absorption rate and the stripping rate were maintained even when the absorption and stripping were repeatedly performed at a relatively high temperature, and thus, the VCM recovery rate was not reduced. In other words, the recovery process using the absorbent of the present invention may increase the VCM stripping speed (recovery speed) and maximize the process efficiency by enabling the recovery process at a high temperature.

Hereinabove, although the present invention has been described by specific matters, limited embodiments, and drawings, they have been provided only for assisting in the entire understanding of the present invention. Therefore, the present invention is not limited to the embodiments. Various modifications and changes may be made by those skilled in the art to which the present invention pertains from this description.

Therefore, the spirit of the present invention should not be limited to the described embodiments, but the claims and all modifications equal or equivalent to the claims are intended to fall within the spirit of the present invention.

### [Detailed Description of Main Elements]

10: Absorption tower
11: Absorption body
30: Stripping tower
31: Stripping body
40: First and second heat exchangers

## Claims

1. A method for recovering an unreacted monomer, the method comprising:
(S1) supplying an exhaust gas discharged from a reactor and containing an unreacted monomer and an absorbent to an absorption unit to allow the absorbent to absorb the unreacted monomer;
(S2) transferring the absorbent into which the unreacted monomer is absorbed to a heat exchange unit and heating the transferred absorbent;
(S3) supplying the heated absorbent and a heating medium to a stripping unit and stripping and recovering the unreacted monomer from the absorbent;
(S4) transferring the absorbent from which the unreacted monomer is stripped to the heat exchange unit and cooling the transferred absorbent; and
(S5) resupplying the cooled absorbent to the absorption unit.

2. The method of claim 1, wherein the absorbent contains a plasticizer.

3. The method of claim 2, wherein the absorbent is derived from the following Chemical Formula 1: 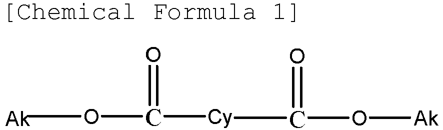 (in Chemical Formula 1, Cy is phenylene or cyclohexylene, and Ak is a C₃ to C₁₀ linear or branched alkyl group).

4. The method of claim 3, wherein the absorbent is dioctyl terephthalate (DOTP) or bis(2-ethylhexyl) cyclohexane-1,4-dicarboxylate (DEHCH).

5. The method of claim 2, wherein the step (S1) is performed at a temperature of 20 to 50°C and a pressure of 1 to 4 barg.

6. The method of claim 2, wherein in the step (S2), a temperature of the absorbent heated by the heat exchange unit is 90 to 120°C.

7. The method of claim 2, wherein in the step (S3), a temperature of the heating medium is 120 to 200°C.

8. The method of claim 2, further comprising, after the step (S1), (Sl-A) supplying and adsorbing a residual unreacted monomer that is not absorbed into the absorbent in the absorption unit to an adsorption unit.

9. The method of claim 2, wherein in the step (S1), each of the exhaust gas and the absorbent is supplied to have a counter-current flow in the absorption unit.

10. The method of claim 2, wherein in the step (S3), each of the absorbent and the heating medium is supplied to have a counter-current flow in the stripping unit.

11. The method of claim 2, wherein the unreacted monomer includes a vinyl chloride monomer (VCM).

12. A device for recovering an unreacted monomer, the device comprising:
an absorption tower to which each of an exhaust gas discharged from a reactor and an absorbent is supplied and in which an unreacted monomer is absorbed into the absorbent;
a first recovery line for recovering the absorbent into which the unreacted monomer is absorbed from the absorption tower;
a first heat exchanger that heats the absorbent supplied from the first recovery line;
a second recovery line for recovering the heated absorbent from the first heat exchanger;
a stripping tower to which each of the absorbent supplied from the second recovery line and a heating medium is supplied and in which the unreacted monomer is stripped from the absorbent;
a first circulation line for recovering the absorbent from which the unreacted monomer is stripped from the stripping tower;
a second heat exchanger that cools the absorbent supplied from the first circulation line; and
a second circulation line for resupplying the cooled absorbent from the second heat exchanger to the absorption tower.

13. The device of claim 12, wherein the absorbent is a plasticizer for a vinyl chloride polymer, and the unreacted monomer is a vinyl chloride monomer (VCM).

14. The device of claim 13, wherein the first and second heat exchangers are integrated.

15. The device of claim 14, wherein the first heat exchanger further includes a heater, and the second heat exchanger further includes a cooler.

16. The device of claim 13, wherein the absorption tower includes an absorption body, a first supply port formed at a lower portion of the absorption body and supplying the exhaust gas into the absorption body, a second supply port located at an upper portion of the absorption body and supplying the absorbent into the absorption body, a first discharge port formed at a bottom of the absorption body and discharging the absorbent into which the unreacted monomer is absorbed, and a second discharge port formed at a top of the absorption body and discharging an unreacted monomer that is not absorbed into the absorbent, and
the stripping tower includes a stripping body, a third supply port formed at a lower portion of the stripping body and supplying the heating medium into the stripping body, a fourth supply port located at an upper portion of the stripping body and supplying the heated absorbent into the stripping body, a third discharge port formed at a bottom of the stripping body and discharging the absorbent from which the unreacted monomer is stripped, and a fourth discharge port formed at a top of the stripping body and discharging the unreacted monomer stripped from the absorbent.

17. The device of claim 13, further comprising an adsorption tower connected to a top of the absorption tower and adsorbing an unreacted monomer that is not absorbed into the absorbent and is discharged from the absorption tower.

18. The device of claim 13, further comprising a third heat exchanger connected to a top of the stripping tower and separating condensed water from the unreacted monomer discharged from the stripping tower.
